# EUROPÄISCHE PATENTANMELDUNG

(11) **EP 4 830 628 A1**
(43) Veröffentlichungstag der Anmeldung: **28.10.2026**
(21) Anmeldenummer: 25172356.5
(22) Anmeldetag: 24.04.2025
(51) Int. Cl.: A61B 5/20, A61B 5/00, G01N 29/024, G01N 33/493

(54) **VERFAHREN ZUR KONTROLLE DER HYDRATION EINES LEBEWESENS**

(71) Anmelder: Sonotec GmbH, 06112 Halle (Saale) (DE); Wiegand, Stefan, 06114 Halle (DE)
(72) Erfinder: WIEGAND, Stefan, 06114 Halle (Saale) (DE); MÜNCH, Hans-Joachim, 06130 Halle (Saale) (DE); CHRISTL, Maik, 06184 Kabelsketal, TO Zwintschöna (DE); KIRCHER, Wolfgang, 06120 Halle (Saale) (DE)
(74) Vertreter: Maikowski & Ninnemann Patentanwälte Partnerschaft mbB

(57) **Zusammenfassung**

Die vorliegende Erfindung stellt ein Verfahren zur Kontrolle der Hydration eines Lebewesens zur Verfügung. Erfindungsgemäß wird
a. die Ultraschalllaufzeit oder
b. die Ultraschallimpulsfrequenz oder
c. die Ultraschallamplitudenänderung und die Ultraschallaufzeit

in einer Urinprobe mit einem Ultraschallsensor gemessen. Aus den Messwerten
• a. oder b. wird die Schallgeschwindigkeit oder
• aus den Messwerten a. oder b. die Schallgeschwindigkeit und aus den Messwerten c. die akustische Impedanz des Urins

berechnet. Zudem wird die Temperatur der Urinprobe gemessen und die Dichte des Urins aus dem Zusammenhang von Temperatur und Schallgeschwindigkeit bestimmt. Der Hydrationszustand des Lebewesens wird anhand der Dichte des Urins bestimmt wird und es erfolgt eine Meldung über den Hydrationszustand. Weiterhin stellt die Erfindung eine Vorrichtung zur Durchführung des Verfahrens zur Verfügung.

## Beschreibung

Die vorliegende Erfindung stellt ein Verfahren zur Kontrolle der Hydration eines Lebewesens zur Verfügung. Erfindungsgemäß wird
a. die Ultraschalllaufzeit oder
b. die Ultraschallimpulsfrequenz oder
c. die Ultraschallamplitudenänderung und die Ultraschallaufzeit
in einer Urinprobe mit einem Ultraschallsensor gemessen. Aus den Messwerten
- a. oder b. wird die Schallgeschwindigkeit oder
- aus den Messwerten a. oder b. die Schallgeschwindigkeit und aus den Messwerten c. die akustische Impedanz des Urins
berechnet. Zudem wird die Temperatur der Urinprobe gemessen und die Dichte des Urins aus dem Zusammenhang von Temperatur und Schallgeschwindigkeit bestimmt. Der Hydrationszustand des Lebewesens wird anhand der Dichte des Urins bestimmt wird und es erfolgt eine Meldung über den Hydrationszustand. Weiterhin stellt die Erfindung eine Vorrichtung zur Durchführung des Verfahrens zur Verfügung.

Allein in Deutschland kam es im Jahr 2021 zu 107.000 Krankenhausbehandlungen und 3.500 Todesfällen aufgrund von akutem Flüssigkeitsmangel und damit einhergehender Dehydration. Die Behandlungsfälle zeigen dabei eine deutliche Korrelation zur Anzahl der Hitzetage in diesem Jahr. Auf Grund des Klimawandels ist davon auszugehen, dass dieses Problem deutlich zunehmen wird.

Die Diagnosestellung der Dehydratation erfolgt bisher anhand des klinischen Bildes, einer Untersuchung des Blutserums und des Urins (insbesondere auf den Natrium-Gehalt, Hämatokrit- und Kreatinin-Werte sowie die Serum- beziehungsweise Urinosmolalität) mit Laboruntersuchungsmethoden. Diese Untersuchungen sind aufwendig und können nur in einer klinischen Umgebung von entsprechendem Fachpersonal durchgeführt werden.

Grund für viele Dehydrierungsfälle ist die unzureichende Aufnahme von Flüssigkeit. Es können also vorbeugende Maßnahmen, nämlich ausreichendes Trinken ergriffen werden, um eine Dehydrierung zu vermeiden. In einigen Bereichen achten die potentiell von einer Dehydrierung betroffenen jedoch von allein nicht ausreichend auf die Trinkmenge oder können dies aus bestimmten Gründen nicht. Beispielsweise in der Pflege ist dies ein wichtiges Thema. Wünschenswert ist es daher den Hydrationszustand mit einfachen Mittel auch außerhalb einer Klinik oder Arztpraxis zu überwachen, um so möglichst zeitig vorbeugende Maßnahmen ergreifen zu können. Hierdurch kann die Lebensqualität der Betroffenen verbessert werden. Zudem kann die Anzahl der Behandlungen in Kliniken oder Arztpraxen aufgrund von akutem Flüssigkeitsmangel reduziert werden, was zu einer Entlastung des Gesundheitssystems führt.

Ziel der vorliegenden Erfindung ist es daher die aus dem Stand der Technik bekannten Nachteile zu beheben und ein Verfahren und eine Vorrichtung bereitzustellen, mit der außerhalb von Kliniken oder Arztpraxen der Hydrationszustand von Lebewesen kontrolliert werden kann.

Hierfür stellt die Erfindung ein Verfahren gemäß dem Anspruch 1 und eine Vorrichtung gemäß dem Anspruch 11 zur Verfügung.

### Detaillierte Beschreibung

Die Erfindung stellt ein Verfahren zur Kontrolle der Hydration eines Lebewesens zur Verfügung.

Lebewesen im Sinne der Erfindung können Menschen oder auch Tiere sein. Bevorzugt wird die Hydration von Menschen mit dem erfindungsgemäßen Verfahren kontrolliert.

Erfindungsgemäß wird
a. die Ultraschalllaufzeit oder
b. die Ultraschallimpulsfrequenz oder
c. die Ultraschallamplitudenänderung und die Ultraschallaufzeit

in einer Urinprobe mit einem Ultraschallsensor gemessen. Aus den Messwerten
- a. oder b. wird die Schallgeschwindigkeit oder
- aus den Messwerten a. oder b. die Schallgeschwindigkeit und aus den Messwerten c. die akustische Impedanz des Urins
berechnet. Weiterhin wird die Temperatur des Urins mit einem Temperatursensor gemessen. Geeignete Temperatursensoren können beispielsweise PT100, PT1000 oder Thermoelemente sein und sind dem Fachmann bekannt.

Die Dichte des Urins wird aus dem Zusammenhang von Temperatur und Schallgeschwindigkeit bestimmt. Hierfür wurde der Zusammenhang zwischen Dichte des Urins, der Temperatur des Urins und der Schallgeschwindigkeit im Urin zuvor experimentell bestimmt. Die experimentellen Daten sind in einer Messelektronik hinterlegt und werden in dem erfindungsgemäßen Verfahren dann für die Zuordnung verwendet.

Durch die Messung der Temperatur der Urinprobe, kann der Einfluss der Temperatur auf die Schallgeschwindigkeit und die Dichte berücksichtigt werden. In einer Ausführungsform erfolgt eine Temperaturkorrektur, indem die Schallgeschwindigkeit auf eine Temperatur von 21°C bezogen auf die Schallgeschwindigkeit von Wasser mit folgender Formel umgerechnet wird. ${c}_{korr} = \frac{c \left(T\right) ∗ {c}_{H 2 O} \left(21°C\right)}{{c}_{H 2 O} \left(T\right)}$ **Wobei**
*c*_{H20}(21°*C*) die Schallgeschwindigkeit in Wasser bei einer Temperatur von 21°C ist;
*c_{H2O}*(*T*) die Schallgeschwindigkeit in Wasser bei der Temperatut T ist;
*c*(*T*) die Schallgeschwindigkeit in der Urinprobe bei der Temperatur T ist.

Der Hydrationszustand des Lebewesens wird anhand der Dichte des Urins bestimmt und eine Meldung über den Hydrationszustand ausgegeben.

Der Zusammenhang zwischen Dichte des Urins und Hydrationszustand wurde zuvor experimentell bestimmt. Diese Daten liegen dem vorliegenden Verfahren zu Grunde und sind in einer Messelektronik hinterlegt. Im einfachsten Fall kann anhand der experimentellen Daten eine Einordung getroffen werden, ob das Lebewesen ausreichend hydriert ist, also ausreichend getrunken hat oder nicht.

Die Berechnung bzw. Bestimmung der Schallgeschwindigkeit und des Hydrationszustandes wird bevorzugt durch eine Messelektronik durchgeführt, die eine geeignete Software aufweist. In der Messelektronik sind die notwendigen bereits beschriebenen experimentellen Daten hinterlegt.

In einer Ausführungsform der vorliegenden Erfindung erfolgt die Meldung über den Hydrationszustand durch
- eine Anzeige auf einer Handy-App und/oder
- per Datenfernübertragung an einen Arzt.

Die Information über den Hydrationszustand kann über eine Handy-App einem Anwender oder einem Arzt angezeigt werden. Die Information über den Hydrationszustand kann aber auch über Datenfernübertragung an einen entfernten Rechner, beispielsweise in einer Arztpraxis übertragen werden. Der Arzt kann so Patientendaten überwachen und in der Diagnostik unterstützt werden. Insbesondere eine Dehydrierung eines Patienten bei unzureichender Flüssigkeitszufuhr kann besser diagnostiziert werden.

In einer Ausführungsform wird der Hydrationszustand aufgezeichnet und/oder eine Handlungsempfehlung mit der Meldung über den Hydrationszustand erfolgt.

Die Aufzeichnung des Hydrationszustandes kann über einen längeren Zeitraum erfolgen, so dass eine Kontrolle des Trinkverhaltens erfolgen kann. Als Resultat dieser Aufzeichnungen kann dem Anwender eine Information gegeben werden, ob er ausreichend trinkt.

In einer Ausführungsform kann eine Handlungsempfehlung an den Anwender ausgegeben werden, die diesem beispielsweise mitteilt, dass eine zusätzliche Aufnahme von Flüssigkeit notwendig ist.

In einer Ausführungsform kann die Meldung des Hydrationszustandes in Form eines Ampelsystems erfolgen. Grün bedeutet hierbei beispielsweise ausreichend getrunken und rot nicht ausreichend getrunken.

In einer Ausführungsform kann die Handlungsanweisung KI unterstützt erzeugt werden. Hierbei kann berücksichtigt werden, dass sich die Dichte des Urins über den Tag verändert und eine hohe Dichte am Morgen zunächst einmal kein Problem darstellt.

In einer Ausführungsform wird die Ultraschalllaufzeit in der Urinprobe mit zwei Ultraschallwandlern gemessen wird, wobei ein Ultraschallwandler als Sender und ein Ultraschallwandler als Empfänger arbeitet und die Ultraschallaufzeit durch eine Urinprobe zwischen Sender und Empfänger durch eine Messelektronik ermittelt wird. Über die Kenntnis der Länge der Messtrecke wird Schallgeschwindigkeit des Urins bestimmt. Eine ergänzende Messung ermittelt die Temperatur der Urinprobe. Aus dem Zusammenhang von Dichte es Urins, Temperatur des Urins und der Schallgeschwindigkeit wird dann die Urindicht bestimmt. In einer Ausführungsform wird eine Vorlaufzeit von der Ultraschallaufzeit abgezogen.

In einer weiteren Ausführungsform wird die Ultraschalllaufzeit in der Urinprobe mit einem Ultraschallwandler und einen Reflektor gemessen, wobei der eine Ultraschallwandler als Sender fungiert, das Ultraschallsignal die Urinprobe durchläuft, durch den Reflektor zum Ultraschallwandler zurückgeworfen wird und von diesem empfangen wird. Der eine Ultraschallwandler wird erfindungsgemäß in dieser Ausführungsform einmal als Sender und unmittelbar danach als Empfänger für das Ultraschallsignal verwendet. Eine Messelektronik ermittelt die Ultraschalllaufzeit zwischen Sender, Reflektor und Empfänger durch die Messtrecke mit der Urinprobe. Aus dem Zusammenhang von Dichte es Urins, Temperatur des Urins und der Schallgeschwindigkeit wird dann die Urindicht bestimmt.

In einer Ausführungsform wird die Ultraschallimpulsfrequenz in einer Urinprobe mit zwei Ultraschallwandlern gemessen.

Ganz allgemein beschreibt die Ultraschallimpulsfrequenz die gesendete Anzahl an Ultraschallimpulsen pro Zeiteinheit.

In der Ausführungsform mit zwei Ultraschallwandlern arbeitet ein Ultraschallwandler als Sender und sendet einen Ultraschallimpuls aus. Der Ultraschallimpuls durchläuft die Urinprobe und wird vom zweiten Ultraschallwandler, der als Empfänger arbeitet, detektiert. Die Urinprobe befindet sich demnach im Schallweg zwischen den beiden Ultraschallwandlern. Beim Eintreffen des Ultraschallimpulses am zweiten Ultraschallwandler wird vom ersten Ultraschallwandler ein weiterer Ultraschallimpuls ausgesendet. Das Eintreffen des Ultraschallimpulses am Empfänger wird durch eine Messelektronik detektiert und initiiert das erneute Aussenden eines Ultraschallimpulses durch den Sender. Durch die Messelektronik wird die gesendete Anzahl an Ultraschallimpulsen pro Zeiteinheit und damit die Ultraschallimpulsfrequenz berechnet. Über die Ultraschallimpulsfrequenz und die Länge der Messstrecke in der Urinprobe kann dann die Schallgeschwindigkeit in der Urinprobe ermittelt werden. Eine ergänzende Messung ermittelt die Temperatur der Urinprobe. Aus dem Zusammenhang von Dichte es Urins, Temperatur des Urins und der Schallgeschwindigkeit wird dann die Urindicht bestimmt.

In einer Ausführungsform wird die Ultraschallimpulsfrequenz in einer Urinprobe mit einem Ultraschallwandler und einem Reflektor gemessen. Der Ultraschallwandler wird elektronisch einmal als Sender und unmittelbar danach als Empfänger verwendet. Der Ultraschallwandler arbeitet zunächst als Sender und sendet einen Ultraschallimpuls aus. Der Ultraschallimpuls durchläuft die Urinprobe und wird vom Reflektor zum Ultraschallwandler zurückreflektiert. Der reflektierte Ultraschallimpuls wird vom Ultraschallwandler detektiert und ein weiterer Ultraschallimpuls wird ausgesendet. Der Ultraschallimpulses durchläuft daher die Messtrecke in der Urinprobe einmal wird am Reflektor reflektiert und durchläuft die Messtrecke in der Urinprobe ein zweites Mal. Nach dem zweimaligen Durchlaufen der Messtrecke wird der Ultraschallimpuls vom Ultraschallwandler, der jetzt als Empfänger arbeitet, detektiert und es wird daraufhin das erneute Aussenden eines Ultraschallimpulses vom Ultraschallwandler, der nun als Sender arbeitet, initiiert. Eine Messelektronik berechnet die gesendete Anzahl an Ultraschallimpulsen pro Zeiteinheit und damit die Ultraschallimpulsfrequenz. Über die Ultraschallimpulsfrequenz und die Länge der Messstrecke in der Urinprobe kann dann die Schallgeschwindigkeit in der Urinprobe ermittelt werden. Eine ergänzende Messung ermittelt die Temperatur der Urinprobe. Aus dem Zusammenhang von Dichte es Urins, Temperatur des Urins und der Schallgeschwindigkeit wird dann die Urindicht bestimmt.

In einer weiteren Ausführungsform wird die Ultraschallamplitudenänderung in einer Urinprobe mit einem Ultraschallwandler mit einer Vorlaufstrecke gemessen. Bevorzugt wird das Material der Vorlaufstrecke so gewählt, dass sich deren akustische Impedanz günstig zur Impedanz von Wasser verhält. Günstig bedeutet, dass die Impedanz des Materials der Vorlaufstrecke möglichst ähnlich zur Impedanz von Wasser ist. Dem Fachmann sind geeignete Kunststoffe bekannt.

Die Vorlaufstrecke ist auf einer ersten Seite in Kontakt mit dem Ultraschallwandler und auf einer zweiten Seite in Kontakt mit der Urinprobe. Der Ultraschallwandler sendet einen Ultraschallimpuls mit einer definierten Amplitude aus. Der Ultraschallimpuls durchläuft die Vorlaufstrecke und trifft auf die Grenzfläche von Vorlaufstrecke und Urinprobe, wobei ein Teil des Ultraschallimpulses reflektiert und ein Teil transmittiert wird. Der reflektierte Teil des Ultraschallimpulses wird vom Ultraschallwandler detektiert und durch eine Messelektronik wird das Amplitudenverhältnis von ausgesendetem Ultraschallimpuls und reflektierten Ultraschallimpuls gebildet und damit die Ultraschallamplitudenänderung. Das Amplitudenverhältnis von ausgesendetem Ultraschallimpuls und reflektiertem Ultraschallimpuls wird durch das Verhältnis der Impedanzen von Vorlaufstrecke und Urinprobe bestimmt. Somit kann aus den Ultraschallimpulsamplituden die akustische Impedanz der akustischen Impedanz der Urinprobe berechnet werden. Zusätzlich wird die Ultraschalllaufzeit nach einer der oben beschriebenen Verfahren gemessen. Aus Messwerten der akustischen Impedanz der Urinprobe und der Ultraschalllaufzeit in der Urinprobe wird über den bekannten Zusammenhang Impedanz-Schallgeschwindigkeit-Dichte die Dicht des Urins ermittelt.

Die akustische Impedanz Z wird wie folgt berechnet: $Z = ρ ∗ c$ und hängt damit von der Dichte *ρ* und der Schallgeschwindigkeit c im Medium ab. Unter Kenntnis der akustischen Impedanz und der Schallgeschwindigkeit in einem Medium kann daher auf die Dichte zurückgeschlossen werden.

In einer weiteren Ausführungsform wird die Ultraschallamplitudenänderung in einer Urinprobe mit einem ersten Ultraschallwandlern mit einer ersten Vorlaufstrecke und einem zweiten Ultraschallwandler mit einer zweiten Vorlaufstrecke gemessen. Alle Merkmale der Vorlaufstrecke die bereits beschrieben wurden gelten für diese Ausführungsform ebenso.

Die erste Vorlaufstrecke ist auf einer ersten Seite in Kontakt mit dem ersten Ultraschallwandler und auf einer zweiten Seite in Kontakt mit der Urinprobe. Die zweite Vorlaufstrecke ist auf einer ersten Seite in Kontakt mit dem zweiten Ultraschallwandler und auf einer zweiten Seite in Kontakt mit der Urinprobe. Die Urinprobe befindet sich demnach im Schallweg zwischen erstem Ultraschallwandler, erster Vorlaufstrecke und zweiter vorlaufstrecke und zweitem Ultraschallwandler. Der erste Ultraschallwandler sendet einen Ultraschallimpuls mit einer definierten Amplitude aus. Der Ultraschallimpuls durchläuft die erste Vorlaufstrecke, die Urinprobe und die zweite Vorlaufstrecke und wird vom zweiten Ultraschallsensor detektiert. Durch eine Messelektronik wird das Amplitudenverhältnis von ausgesendetem Ultraschallimpuls und detektierten Ultraschallimpuls gebildet und damit die Ultraschallamplitudenänderung. Das Amplitudenverhältnis von ausgesendetem Ultraschallimpuls und reflektiertem Ultraschallimpuls wird durch das Verhältnis der Impedanzen von Vorlaufstrecke und Urinprobe bestimmt. Somit kann aus den Ultraschallimpulsamplituden die akustische Impedanz der akustischen Impedanz der Urinprobe berechnet werden. Zusätzlich wird die Ultraschalllaufzeit nach einer der oben beschriebenen Verfahren gemessen. Aus Messwerten der akustischen Impedanz der Urinprobe und der Ultraschalllaufzeit in der Urinprobe wird über den bereits beschriebenen Zusammenhang Impedanz-Schallgeschwindigkeit-Dichte die Dicht des Urins ermittelt.

In einer Ausführungsform wird zusätzlich zur Dicht des Urins auch dessen Gesamtvolumen bestimmt. Die Messung des Gesamtvolumens kann beispielsweise über eine Integration einer Volumenstrommessung (z.B. mittels ultraschallbasierter Laufzeitmessung) durchgeführt werden. Dieses Verfahren ist dem Fachmann bereits bekannt und wird beispielsweise in der EP 4 344 637 A1 beschrieben. Die Messung des Gesamtvolumens kann die Diagnostik durch einen Arzt mit wertvollen Informationen unterstützen.

In einer weiteren Ausführungsform der Erfindung wird zusätzlich die Häufigkeit des Wasserlassens dokumentiert. Dies kann durch eine geeignete Software in der Messelektronik durchgeführt werden. Auf diese Weise kann mit Hilfe der vorliegenden Erfindung ein patientenfreundliches Miktionsprotokoll erstellt werden.

Die Erfindung stellt weiterhin eine Vorrichtung zur Kontrolle der Hydration eines Lebewesens zur Verfügung. Die erfindungsgemäße Vorrichtung weist
- eine Messeinheit mit einem Ultraschallsensor aufweisend mindestens einen Ultraschallwandler und eine Messelektronik;
- ein Gehäuse;
- einen Trichter;
auf, wobei die Messelektronik eine Verbindung zu einem externen Gerät aufweist und die Vorrichtung dazu eingerichtet ist, ein Verfahren gemäß der vorliegenden Erfindung durchzuführen.

Ein externes Gerät im Sinne der vorliegenden Erfindung kann eine Recheneinheit wie ein Computer, Tablet oder Smartphone sein.

Der Trichter wird so an der Messeinheit angebracht, dass eine Urinprobe im Trichter gesammelt werden kann und der Messstrecke in der Messeinheit zugeführt wird. Die gesamte Vorrichtung befindet sich in einem Gehäuse, so dass von außen nur der Trichter zum Einleiten einer Urinprobe zugänglich ist. Gespült wird die Vorrichtung mithilfe einer Reinigungsflüssigkeit. Entleert werden kann die Vorrichtung durch einfaches Auskippen über den Trichter. Die Vorrichtung ist damit vom Anwender sehr einfach bedienbar.

In einer Ausführungsform ist die Verbindung zu einem externen Gerät eine Internetverbindung.

Sämtliche Merkmale, die für das erfindungsgemäße Verfahren beschrieben wurden, treffen genauso auf die erfindungsgemäße Vorrichtung zu und andersherum.

Vorteilhafterweise ermöglicht die vorliegende Erfindung einen Einsatz im Home Care Bereich, da die Anwendung des Verfahrens einfach und kostengünstig ist.

Die vorliegende Erfindung stellt eine Möglichkeit dar, um zu überwachen, ob Lebewesen, insbesondere Menschen ausreichend trinken. Dies ist eine präventive Maßnahme gegen Dehydrierung und damit verbundene Folgeerkrankungen.

Die vorliegende Erfindung kann im Bereich
- der Pflege;
- des Leistungssports;
- im Life-Style Bereich;
- im Bereich der Diagnostik von Erkrankungen (beispielsweise Diabetes insipidus); eingesetzt werden.

Vorteilhafterweise ermöglicht die Erfindung
- die Kontrolle des Toilettengangs zum Wasserlassen (Häufigkeit, Menge).
- die Qualitative Kontrolle des Urins hinsichtlich Dichte zur Bestimmung zwischen einer ausreichenden und unzureichenden Wasseraufnahme.
- das Monitoring der Häufigkeit des Wasserlassens (Erstellung eines patientenfreundlichen Miktionsprotokolls, was bei der Diagnostik z.B. von neurogenen Blasenfunktionsstörungen wichtig ist).

Im Folgenden wird die Erfindung anhand von zwei Figuren und einem Ausführungsbeispiel näher beschrieben.
- Figur 1: A bis D zeigen Ausschnitte einer erfindungsgemäßen Vorrichtung;
- Figur 2: stellt den experimentellen Zusammenhang zwischen Schallgeschwindigkeit und Dichte dar.

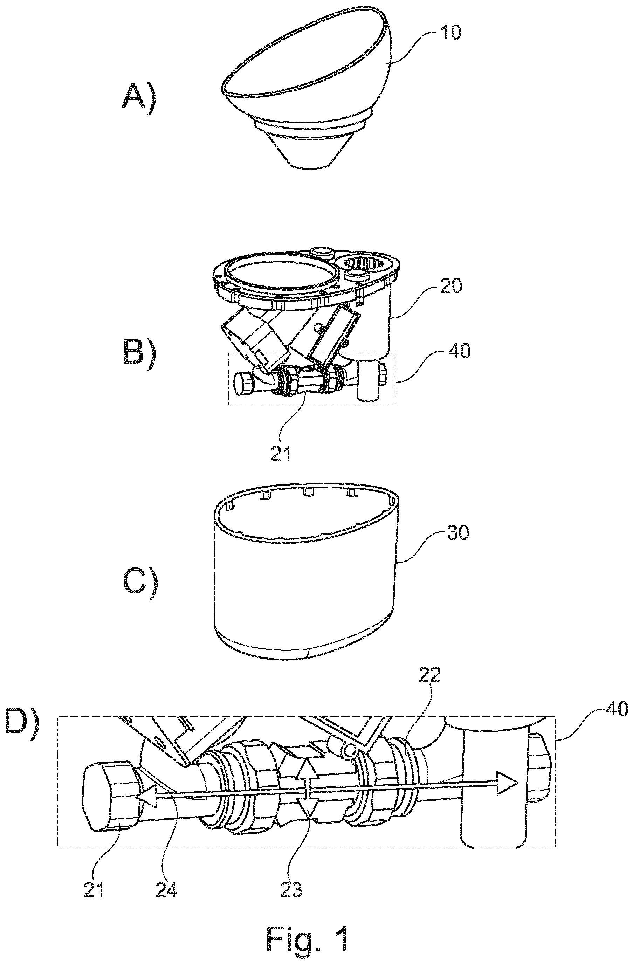

Figuren 1A bis D zeigen Ausschnitte einer erfindungsgemäßen Vorrichtung. Figur 1A zeigt einen Trichter 10 der zum Sammeln der Urinprobe verwendet werden kann. Figur 1B zeigt die Messeinheit 20 mit dem Ultraschallsensor 21. Messtrecken können im Ultraschallsensor 21 beispielsweise längs 24 des Ultraschallsensors oder auch quer 23 zur Flussrichtung der Urinprobe genutzt werden. Im Ultraschallsensor ist ein Temperatursensor 22 integriert. Siehe Figur 1D, die den Ausschnitt 40 der Messeinheit 20 zeigt. Figur 1C zeigt das Gehäuse der erfindungsgemäßen Vorrichtung.

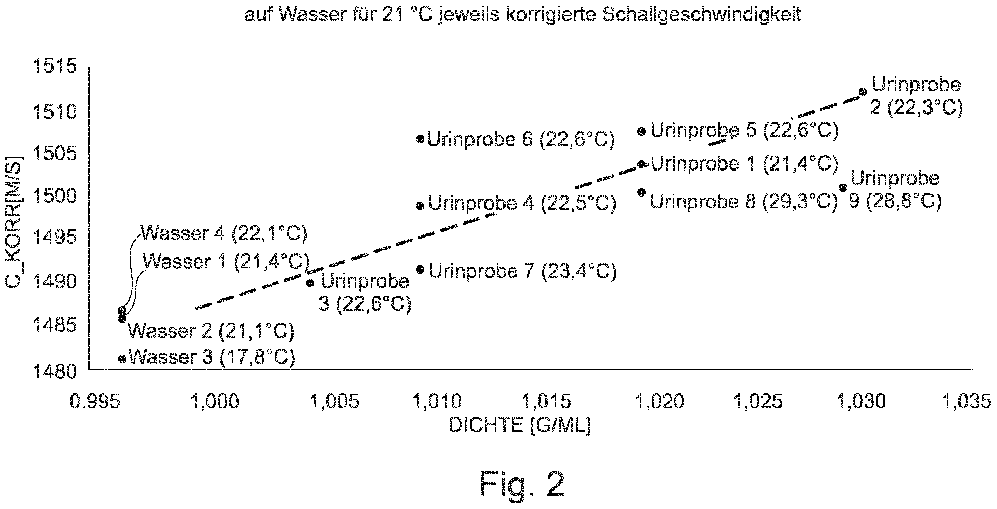

Figur 2 stellt den experimentellen Zusammenhang zwischen Schallgeschwindigkeit und Dichte dar. Figur 2 wird im Ausführungsbeispiel näher beschrieben.

### Ausführungsbeispiel

Die Ultraschallaufzeit wurde von verschiedenen Urinproben gemessen und aus dieser die Schallgeschwindigkeit in der Urinprobe berechnet. Für sämtliche Messwerte wurde eine Temperaturkorrektur vorgenommen, indem die Schallgeschwindigkeit auf eine Temperatur von 21°C bezogen auf die Schallgeschwindigkeit von Wasser umgerechnet wurde. ${c}_{korr} = \frac{c \left(T\right) ∗ {c}_{H 2 O} \left(21°C\right)}{{c}_{H 2 O} \left(T\right)}$ **Wobei**
*c*_{H20}(21°*C*) die Schallgeschwindigkeit in Wasser bei einer Temperatur von 21°C ist;
*c_{H2O}*(*T*) die Schallgeschwindigkeit in Wasser bei der Temperatut T ist;
*c*(*T*) die Schallgeschwindigkeit in der Urinprobe bei der Temperatur T ist.

Die Dichte der Urinproben wurde mit einem Teststreifen gemessen und die korrigierten Schallgeschwindigkeiten *cₖₒᵣᵣ* wurden gegen die Dichte aufgetragen. Das Ergebnis ist in Figur 2 dargestellt. Deutlich zeigt sich eine Abhängigkeit Schallgeschwindigkeit von der Dichte der Urinprobe. Analoge experimentelle Daten werden im erfindungsgemäßen Verfahren verwendet, um die Dichte der Urinprobe für eine bestimmte Temperatur mit Hilfe der Schallgeschwindigkeit zu berechnen. Entsprechende experimentelle Daten sind in der Messelektronik hinterlegt.

### Bezugszeichenliste

- 10: Trichter
- 20: Messeinheit
- 21: Ultraschallsensor
- 22: Temperatursensor
- 23, 24: Messstrecke
- 30: Gehäuse

## Patentansprüche

1. Verfahren zur Kontrolle der Hydration eines Lebewesens, **dadurch gekennzeichnet, dass**
a. die Ultraschalllaufzeit oder
b. die Ultraschallimpulsfrequenz oder
c. die Ultraschallamplitudenänderung und die Ultraschallaufzeit in einer Urinprobe mit einem Ultraschallsensor gemessen wird;
die Schallgeschwindigkeit aus den Messwerten a. oder b. berechnet wird oder die Schallgeschwindigkeit aus den Messwerten a. oder b. und die akustische Impedanz des Urins aus den Messwerten c. berechnet wird;
die Temperatur des Urins gemessen wird;
die Dichte des Urins aus dem Zusammenhang von Temperatur und Schallgeschwindigkeit bestimmt wird;
der Hydrationszustand des Lebewesens anhand der Dichte des Urins bestimmt wird; und eine Meldung über den Hydrationszustand erfolgt.

2. Verfahren gemäß Anspruch 1, **dadurch gekennzeichnet, dass** die Meldung über den Hydrationszustand durch
• eine Anzeige auf einer Handy-App erfolgt und/oder
• per Datenfernübertragung an einen Arzt erfolgt.

3. Verfahren gemäß einem der Ansprüche 1 oder 2, **dadurch gekennzeichnet, dass** der Hydrationszustand aufgezeichnet wird und/oder dass eine Handlungsempfehlung mit der Meldung über den Hydrationszustand erfolgt.

4. Verfahren gemäß einem der Ansprüche 1 bis 3, **dadurch gekennzeichnet, dass** die Ultraschalllaufzeit in der Urinprobe mit zwei Ultraschallwandlern gemessen wird, wobei ein Ultraschallwandler als Sender und ein Ultraschallwandler als Empfänger arbeitet und die Ultraschallaufzeit durch eine Urinprobe zwischen Sender und Empfänger durch eine Messelektronik ermittelt wird.

5. Verfahren gemäß Anspruch 4, **dadurch gekennzeichnet, dass** weiterhin eine Vorlaufzeit von der Ultraschallaufzeit abgezogen wird.

6. Verfahren gemäß einem der Ansprüche 1 bis 3, **dadurch gekennzeichnet, dass** die Ultraschalllaufzeit in der Urinprobe mit einem Ultraschallwandler und einen Reflektor gemessen wird, wobei der eine Ultraschallwandler als Sender fungiert, das Ultraschallsignal die Urinprobe durchläuft, durch den Reflektor zum Ultraschallwandler zurückgeworfen wird und von diesem empfangen wird.

7. Verfahren gemäß einem der Ansprüche 1 bis 3, **dadurch gekennzeichnet, dass** die Ultraschallimpulsfrequenz in einer Urinprobe mit zwei Ultraschallwandlern gemessen wird, wobei
ein Ultraschallwandler als Sender arbeitet und einen Ultraschallimpuls aussendet; der Ultraschallimpuls die Urinprobe durchläuft und vom zweiten Ultraschallwandler, der als Empfänger arbeitet, detektiert wird;
beim Eintreffen des Ultraschallimpulses am zweiten Ultraschallwandler vom ersten Ultraschallwandler ein weiterer Ultraschallimpuls ausgesendet wird;
durch eine Messelektronik die gesendete Anzahl an Ultraschallimpulsen pro Zeiteinheit und damit die Ultraschallimpulsfrequenz berechnet wird.

8. Verfahren gemäß einem der Ansprüche 1 bis 3, **dadurch gekennzeichnet, dass** die Ultraschallimpulsfrequenz in einer Urinprobe mit einem Ultraschallwandler und einem Reflektor gemessen wird, wobei
der Ultraschallwandler als Sender arbeitet und einen Ultraschallimpuls aussendet; der Ultraschallimpuls die Urinprobe durchläuft und vom Reflektor zum Ultraschallwandler reflektiert wird;
der reflektierte Ultraschallimpuls vom Ultraschallwandler detektiert wird und ein weiterer Ultraschallimpuls ausgesendet wird;
durch eine Messelektronik die gesendete Anzahl an Ultraschallimpulsen pro Zeiteinheit und damit die Ultraschallimpulsfrequenz berechnet wird.

9. Verfahren gemäß einem der Ansprüche 6 bis 8, **dadurch gekennzeichnet, dass** weiterhin die Ultraschallamplitudenänderung in einer Urinprobe mit einem Ultraschallwandler mit einer Vorlaufstrecke gemessen wird, wobei die Vorlaufstrecke auf einer ersten Seite in Kontakt mit dem Ultraschallwandler ist und auf einer zweiten Seite in Kontakt mit der Urinprobe;
der Ultraschallwandler einen Ultraschallimpuls mit einer definierten Amplitude aussendet;
der Ultraschallimpuls die Vorlaufstrecke durchläuft und auf die Grenzfläche von Vorlaufstrecke und Urinprobe trifft, wobei ein Teil des Ultraschallimpulses reflektiert und ein Teil transmittiert wird;
der reflektierte Teil des Ultraschallimpulses wird vom Ultraschallwandler detektiert;
durch eine Messelektronik wird das Amplitudenverhältnis von ausgesendetem Ultraschallimpuls und reflektierten Ultraschallimpuls gebildet und damit die Ultraschallamplitudenänderung.

10. Verfahren gemäß einem der Ansprüche 6 bis 8, **dadurch gekennzeichnet, dass** weiterhin die Ultraschallamplitudenänderung in einer Urinprobe mit einem ersten Ultraschallwandlern mit einer ersten Vorlaufstrecke und einem zweiten Ultraschallwandler mit einer zweiten Vorlaufstrecke gemessen wird, wobei die erste Vorlaufstrecke auf einer ersten Seite in Kontakt mit dem ersten Ultraschallwandler ist und auf einer zweiten Seite in Kontakt mit der Urinprobe;
die zweite Vorlaufstrecke auf einer ersten Seite in Kontakt mit dem zweiten Ultraschallwandler ist und auf einer zweiten Seite in Kontakt mit der Urinprobe;
der erste Ultraschallwandler einen Ultraschallimpuls mit einer definierten Amplitude aussendet;
der Ultraschallimpuls die erste Vorlaufstrecke, die Urinprobe und die zweite Vorlaufstrecke durchläuft und vom zweiten Ultraschallsensor detektiert wird;
durch eine Messelektronik wird das Amplitudenverhältnis von ausgesendetem Ultraschallimpuls und detektierten Ultraschallimpuls gebildet und damit die Ultraschallamplitudenänderung.

11. Vorrichtung zur Kontrolle der Hydration eines Lebewesens, **dadurch gekennzeichnet, dass** die Vorrichtung
• eine Messeinheit mit einem Ultraschallsensor aufweisend mindestens einen Ultraschallwandler und eine Messelektronik;
• ein Gehäuse;
• einen Trichter;
aufweist, wobei die Messelektronik eine Verbindung zu einem externen Gerät aufweist und die Vorrichtung dazu eingerichtet ist, eine Verfahren gemäß einem der Ansprüche 1 bis 10 durchzuführen.

12. Vorrichtung gemäß Anspruch 11, **dadurch gekennzeichnet, dass** die Verbindung zu einem externen Gerät eine Internetverbindung ist.
